# EUROPEAN PATENT SPECIFICATION

(11) **EP 2 041 254 B1**
(45) Date of publication and mention of the grant of the patent: **07.11.2012**
(21) Application number: 07787172.1
(22) Date of filing: 06.07.2007
(51) Int. Cl.: A61K 8/40, C11D 3/00, C11D 3/20, C11D 3/39, C11D 17/04, A01N 25/16, A01N 31/02, A01N 59/00, A61Q 19/02, A61K 8/34, A61K 8/22, A61K 8/44, A61K 8/46, A61Q 17/00, A61K 8/04, A61K 8/39, C11D 3/48

(54) **BROAD SPECTRUM AND SKIN FRIENDLY DISINFECTING COMPOSITION**
HAUTFREUNDLICHES DESINFEKTIONSMITTEL MIT BREITEM SPEKTRUM
COMPOSITION À LARGE SPECTRE D'EFFICACITÉ ET DE DÉSINFECTION DOUCE DE LA PEAU

(30) Priority: 06.07.2006 EP 06116742
(43) Date of publication of application: 01.04.2009
(73) Proprietor: Centennial Ventures BV, 1213 RT Hilversum (NL)
(72) Inventor: BOBBERT, Ilja, 1213 RT Hilversum (NL)
(74) Representative: van Heuvel, Margaretha
(86) International application number: PCT/EP2007/056889
(87) International publication number: WO 2008/003779

(56) References cited:
- WO-A-2005/030917
- US-A- 3 954 974
- US-A- 4 900 721
- US-A- 6 086 856

## Description

The present invention is directed to ready-to-use skin friendly, broad spectrum disinfectant compositions which include both an alcohol and hydrogen peroxide as active disinfecting constituents.

Infection control is a major concern for health care professionals. Viruses and bacteria on contaminated hands are easily spread among people in health care facilities such as hospitals. Of course, the risk of infection is also present in public places other than hospitals, such as in gyms, washrooms, restaurants, and schools.

A recent trend exists in hospital settings and in food & hospitality sectors towards higher levels of infection control. In addition, an increasing awareness exists for infectious diseases that can be transferred via the skin and respiratory system. These trends have opted the industry to come up with biocidal solutions that can be used more frequently throughout the day. These solutions must be hypoallergenic, non toxic and not produce any residue on the skin that is undesirable.

In certain environments, such as hospitals, the required level of disinfection cannot be achieved by most of the commonly available products. Consequently, specific hand disinfectants have been developed to achieve higher levels of disinfection where the need exists. These types of products generally contain antimicrobial active ingredients like alcohols in high concentrations, iodines/iodophors, chlorhexidine gluconate (CHG), phenolic compounds like parachlorometaxylenol (PCMX) and Triclosan, quaternary ammonium compounds or combinations thereof.

A problem with such products is that they often sacrifice skin mildness for the sake of disinfectant activity or vice versa. For example, while raising the concentration of the active ingredient may lead to a higher level of disinfection, it frequently leads to increased skin irritation.

Parachlorometaxylenol (PCMX) and Triclosan are common phenolic compounds used in antiseptic hand wash solutions. Although PCMX and Triclosan have lower toxicity than other phenols, and are rather mild to the skin, their germicidal activity is low and depends on the formulation ingredients.

Iodine and iodophors have been used in antiseptic hand wash formulations for a long time. Their germicidal activities are low and reduced in the presence of organic matter. Furthermore, these ingredients are toxic and can irritate and stain the skin.

Chlorhexidine gluconate (CHG) is used as a skin cleanser, pre-surgical scrub, germicidal hand rinse and wound cleaner. It is less effective against gram-negative bacteria as compared to gram-positive bacteria and exhibits relatively low germicidal activity.

Alcohols represent a well known group of effective germicides, providing rapid disinfection. They also enhance drying of the disinfecting solution on surfaces or while rubbing the hands, providing for a no-rinse, leave-to-dry application. They also provide for a refreshed feeling on the skin. However, in order to achieve sufficiently high disinfection rates, the disinfecting solution must contain at least 60-70% of the alcohol. These levels of alcohols extremely defat and dry the skin, causing a dry, chapped or cracked skin on repeated use. Damaged skin may even increase the chance of bacterial contamination and presence of bacterial residues, because cracks in the skin are difficult to reach and clean properly. Furthermore, the anti-microbial activity of alcohols tends to drop dramatically when used on wet hands, resulting in insufficient germ kill.

To overcome some of these disadvantages of alcohols, it is known to include additional ingredients in the composition, such as emollients, humectants, and surfactants. For example, US patent 6,617,294 discloses a waterless disinfecting hand cleanser made of a combination of 60 to 90% w/w of an alcohol, silicone based materials, and humectants. However, these solutions provide for a sticky residue and cannot be used frequently during the day because of the unwanted and unpleasant build up of the silicone materials.

US 5,916,568 discloses a flash-dry disinfectant composition for use as a hand wash comprising 55-80% of a C2-C5 alcohol, 15-35% of a hydrogen peroxide solution providing an effective hydrogen peroxide concentration of about 1.5-3.5% and 5-10% of a bacteriostatic skin emollient. The high alcohol concentration is biocidal and allows quick drying but also causes severe defatting of the skin, especially after repeated use during the day. Also a hydrogen peroxide concentration in the higher ranges, around 2.5-3.5%, is preferred. However, such concentrations bleach and irritate the skin, especially after repeated use during the day. Therefore, this composition is not particularly useful for frequent use on the skin. Furthermore, high alcohol levels are flammable and therefore give rise to safety risks in use and storage.

US 4,900,721 discloses an aqueous disinfectant for disinfecting the skin and mucous membranes comprising 8-25% of a C2-C8 alcohol and 0.2-0.7% hydrogen peroxide. To be sufficiently biocidal, this composition requires the presence of additional biocidal phenolic compounds, quaternary ammonium compounds and nitrogen-containing biguanide compounds like Chlorhexidine or Vantocil. The latter compounds are undesirable to be used on the skin, especially with more frequent use when build up occurs. Several of the phenolic compounds are also well known as skin allergens, especially when the composition is repeatedly used throughout the day. In particular, it would be advantageous to avoid the use of quaternary ammonium or phenolic compounds, while still reaching the required disinfection rate at sufficiently short contact times.

US 6,106,774 discloses a ready to use aqueous hard surface cleaning and disinfecting composition comprising 0.1-20% of a C1-6 alcohol and 0.1-10% hydrogen peroxide. The composition further should include 1.0-10% of a glycol ether, or butoxypropanol or propoxypropanol, to provide the desirable stain and soil solubilizing effect. However, the lower ranges of the percentage hydrogen peroxide, i.e. ranges that are suitable for skin application, do not provide a suitably fast disinfection in combination with the higher ranges of the percentage alcohol.

US 3,954,974 discloses an oil-in-water-emulsion for disinfecting the skin. The emulsion comprises hydrogen peroxide and, optionally, ethanol in the aqueous phase. The components present in the oil phase have the disadvantage of producing a sticky residue. In addition, such emulsions do not produce a proper foam.

WO 2005/030917 discloses a foaming high alcohol content composition comprising at least 40% of a C2-C4 alcohol (in the examples at least 50% alcohol is used), an antimicrobial agent and a fluorosurfactant to produce a foaming composition. Compositions with such a high alcohol content were shown to require a fluorosurfactant to produce a foam, whereas other surfactants like alkylglucosides or betaines did not produce a foam. Even though they are able to produce a flash foam, the use of fluorosurfactants on the skin is less desirable. In addition, fluorosurfactants are environmentally unfriendly compounds.

Thus, it is highly desirable to avail of a composition for use on the skin and/or mucous membranes that is able to disinfect within a few minutes, that does not need rinsing after use (thus is suitable for leave-on use), that enables drying by rubbing hands and/or arms, and that is skin friendly in that it does not produce stickiness after repeated use, does not cause irritation of the skin and/or is not defattening.

In a first aspect, the present invention provides a biocidal composition as defined in claim 1 for disinfecting skin and/or mucous membranes comprising a C2-C6 alcohol, hydrogen peroxide and a foam booster. It appeared to be highly advantageous to apply the biocidal composition in the form of a foam, in order to provide for exact dosing, to increase contact surface and contact time, and thus to provide a sufficient biocidal efficacy, and to avoid spilling or dripping of the applied dose off the hands, as may be the case in for instance a spray formulation.

The foamable composition of the invention is in the form of an aqueous solution and does not encompass oil-in-water emulsions.

Thus, the composition of the invention is suitable to produce an instant foam when mixed with air using a foam dispenser. Any foam dispenser known in the art is usable, either a pressurized (with a gas propellant) or a non-pressurized system (air driven). In case of a non-pressurized foam dispenser, the foam is produced by pushing the liquid through a membrane whereby the liquid is mixed with air in an air/liquid mixing chamber enabling the production of instant foam from about 0.2 - 3 ml of liquid per push. The liquid is mixed with air in an air to liquid ratio of about 6 to 20, preferably 8 to 15, more preferably 9 to 11. In this way, a relatively "dry" foam is produced.

The mixing (foaming) chamber that contains and dispenses the composition may be an air-driven, that is, the foam-producing gas consists essentially of air. The foam produced by such a device is an aerated foam. A preferred foam dispensing device comprises a reversibly compressible and decompressible contained housing which operates at atmospheric pressure. Suitable foam dispensing devices are produced by Rexam Airspray and Keltec Dispensing Systems which deliver creamy, high-quality foam without the use of non-air propellants.

An overview of suitable foam dispenses from Rexam Airspray is provided in the table below:

| **Type** | **Air to Liquid Ratio** | **Output ml** | **Stroke mm** |
|---|---|---|---|
| L9 | 9 | 1.20 | 18.8 |
| S10 | 10 | 0.40 | 11.0 |
| L11 | 11 | 0.75 | 14.8 |

The composition of the invention comprises 0.1-5% hydrogen peroxide, 26-50% of a C2-C6 alcohol, and 0.01-2% of a foam booster.

Throughout the present invention, percentages are expressed as weight percentages based on total weight of the composition, unless indicated otherwise.

The compositions of the invention comprises a C2-C6 alcohol, preferably a C2-C4 alcohol such as ethanol, propanol, isopropanol, butanol or 1,3-butanediol, or a mixture thereof, more preferably ethanol, the alcohol being present in a concentration (w/w) of 26-50%, preferably of 30-45%, most preferably 30-40%.

The composition comprises hydrogen peroxide as a secondary disinfecting agent. The hydrogen peroxide is provided in an amount of 0.1-5%, preferably 0.2-3%, more preferably 0.2-2%, even more preferably 0.5-2%, most preferably 0.5-1.5% (w/w). A minimum of 0.5% (w/w) hydrogen peroxide typically is required to achieve a hospital strength disinfection level. Higher concentrations than 3% are generally to be avoided when using repeatedly during the day, as it has been observed that such levels may cause skin bleaching.

The composition of the invention further comprises a foam booster for providing a stable foam in the presence of an alcohol, when dispensing the composition from a foam dispenser.

In the context of the invention, the term "foam booster" refers to a compound that is able to produce a stable foam in the presence of concentrations of alcohols as mentioned herein.

Preferably, the foam booster used in the composition of the invention provides a stable foam when dispensing a composition consisting of 36% (w/w) ethanol and 0.2% (w/w), preferably 0.3% (w/w), of the foam booster from a foam dispenser with a relatively high air to liquid ratio, preferably an air to liquid ratio of 9-11, more preferably an air to liquid ratio of 11, on an inert and smooth surface at about 20 °C. A suitable foam pump is for instance the L11 foam pump from Rexam Airspray, which has an air to liquid ratio of 11, a liquid output volume of 0.75 ml, a stroke of 14.8 mm. The foam is optimally produced by applying regular speed and pressure onto the foam dispenser, as is known to one skilled in the art.

According to this invention, a stable foam is a foam that remains as a foam for at least 5-10 seconds, preferably for at least 10-15 seconds, more preferably for at least 15-20 seconds, even more preferably for at least 20-25 seconds, most preferably for at least 25-30 seconds, as measured immediately after dispensing from a foam dispenser as mentioned above.

The foam booster should be suitable for use on the skin or mucous membranes, in particular suitable for a leave-on application, i.e. without rinsing after its application. The foam booster preferably does not cause skin irritation or an allergic reaction.

The present invention thus provides compositions with a relatively high content of lower alcohols (C2-C6), able to be dispensed as a foam and being able to disinfect the skin and not causing any unwanted residues after drying of the skin. The foamable compositions when mixed with air deliver a stable foam which can be used for personal cleaning and disinfecting purposes and which breaks on pressure application such as when a user rubs his hands or when the foam after application is rubbed over a surface. Depending on the alcohol concentration and the concentration and type of foam booster, the foam produced can vary widely, from relatively fast breaking but stable enough to be properly applied onto the skin without waste, up to a long-lasting foam.

The foam booster is present in a concentration of 0.01-2%, preferably, 0.02-1.5%, more preferably 0.05-1%. The concentration of the foam booster will depend among others on the water and alcohol content of the composition. Generally, an increase in alcohol concentration also requires an increase in foam booster concentration, in order to get a stable foam. However, the foam booster concentration should typically be as low as possible in order to avoid residue build up on the hands, causing stickiness with repeated use. Also, the foam booster should be stable in the presence of alcohol and hydrogen peroxide.

The foam booster is chosen from at least one of i) an alkoxylated non-ionic surfactant, ii) a nitrogen containing amphoteric or non-ionic surfactant, iii) a linear C12-C24 alpha olefin sulfonate, and iv) a C12-C20 sulfosuccinate.

In one embodiment, the foam booster is not chosen from the group of fluorosurfactants as disclosed in WO 2005/030917.

A particularly preferred foam booster is chosen from i) alkoxylated non-ionic surfactants and/or ii) nitrogen containing amphoteric or non-ionic surfactants.

The foam booster may preferably be chosen from the group of oil-in-water emulsifiers and solubilisers, such as typically found in the group of alkoxylated non-ionic surfactants as described herein.

The alkoxylated non-ionic surfactant preferably is an alkoxylated C8-C30 fatty alcohol, fatty acid and/or fatty oil, all with a degree of alkoxylation of minimal 6, and/or a polysorbate.

The alkoxylated C8-C30 fatty alcohol comprises a fatty alcohol component that is a primary and/or secondary alcohol, preferably a linear primary and/or secondary alcohol, more preferably a linear primary alcohol. Preferred examples of the above C8-C30 fatty alcohols are C10-C20 fatty alcohols. The alkoxylated fatty alcohol further has a degree of alkoxylation of at least 6, preferably at least 7, more preferably has a degree of alkoxylation of 7-14. A preferred alkoxylated fatty alcohol is a C12-C18 fatty alcohol ethoxylate with 7 moles of ethylene oxide, such as Cognis Dehydol-LT 7.

The alkoxylated C8-C30 fatty oil preferably comprises a hydrogenated fatty oil component. More preferably, it is an alkoxylated hydrogenated C10-C30 fatty oil, with preferably an alkoxylation degree of at least 20, more preferable 20-60. Examples are alkoxylated hydrogenated castor oil, alkoxylated hydrogenated coco oil, alkoxylated hydrogenated palm oil, and alkoxylated hydrogenated olive oil. Particularly preferred alkoxylated hydrogenated fatty oil non-ionic surfactants are Eumulgin HRE 40 of Cognis or Cremophor CO of BASF.

The alkoxylated C8-C30 fatty acid has an alkoxylation degree of preferably 6-12. Examples are ethoxylated caprate, ethoxylated caprylate, ethoxylated cocoate, etc.

Further advantageous alkoxylated non-ionic surfactants are polysorbates. Polysorbates are sorbitol based emulsifiers. Preferred polysorbates are polysorbate 20, 40, 60 or 80, such as the Tween series of Uniqema and Eumulgin SML, SMO and SMS of Cognis.

The alkoxylated non-ionic surfactants as mentioned above preferably are ethoxylated surfactants.

These alkoxylated, preferably ethoxylated, non-ionic surfactants may advantageously also possess a high wetting capacity. The wetting capacity of a surfactant is the capacity to lower the surface tension of water significantly, thereby enhancing the spreading of the aqueous composition over the skin and improving drying of the skin by reducing the drying time. Because the composition of the invention also contains a significant amount of water, the addition of a foam booster that also has high wetting capacity is advantageous to enhance drying of the skin.

When aiming at increased skin tolerance and wetting capacity of the alkoxylated non-ionic surfactants, compounds with a longer alkyl carbon chain, preferably at least a C12, more preferably at least a C14 chain, and a higher alkoxylation degree may be chosen.

The nitrogen containing amphoteric or non-ionic surfactant preferably is an Amine Oxide, Betaine, Amide, Hydroxysultaine and/or Imidazoline derivative. It comprises at least one amine oxide, betaine, hydroxysultaine, and/or amide group and/or is an imidazoline derivative, and further comprises a straight carbon chain containing at least 8 carbon atoms, preferably at least 10 carbon atoms, more preferably 10-18 carbon atoms, most preferably 12-18 carbon atoms, attached to the nitrogen. The straight carbon-containing chain may be a fully saturated alkyl or acyl chain, or may be a carbon chain containing a double bond and/or a carbon chain interrupted by a nitrogen and/or oxygen atom, for instance an (additional) amide group. The carbon-containing chain may optionally contain a few (e.g. 1-3) methyl or ethyl substituents. The amine oxides and betaines further contain one or two short chain alkyl groups attached to the nitrogen, preferably ethyl and/or methyl.

These nitrogen containing amphoteric or non-ionic surfactants advantageously may also possess biocidal activity and act synergistically with the hydrogen peroxide and/or alcohol present in the composition.

Preferred amine oxides are C8-C18, preferably C10-C18, more preferably C12-C18 alkyl dimethyl amine oxides, such as decyl dimethylamine oxide, lauryl dimethylamine oxide, myristyl dimethylamine oxide, and/or C10-C18, preferably C12-C18 alkyl amidopropyldimethylamine oxides, such as cocamidopropylamine oxide. Preferred betaines are C10-C18 alkyl dimethyl betaines, such as lauryl betaine, and/or C10-C18 alkyl amidopropyl dimethyl betaines, such as capryl/capramidopropyl betaine, cocamidopropyl betaine, and/or oleamidopropyl betaine. Preferred hydroxysultaines are C10-C18 alkyl hydroxysultaine, such as lauryl hydroxysultaine or cocamidopropyl hydroxysultaine. Preferred amides are C10-C18 alkyl monoethanol, diethanol and/or triethanol amides, such as cocamide MEA, cocamide DEA and/or cocamide TEA, and/or ethoxylated alkylene amides, such as PEG4 rapeseedamide. Preferred imidazoline derivatives are C10-C18 imidazoline deprived amphoteric surfactants, such as sodium cocoamphoacetate, sodium lauroamphoacetate, disodium cocoamphodiacetate, disodium cocoamphodipropionate, and/or disodium lauroamphodiacetate.

A suitable linear C12-C24 alpha olefin sulfonate, is found in Hansa Croup's Hansanil OS (Sodium Olefin C14-C16 Sulfonate).

A suitable Sulfosuccinate is a longer chain (C12-C20) amido type sulfosuccinates, such as cocamido MEA or MIPA Sulfosuccinate, oleamido MEA or MIPA Sulfosuccinate, and the C12-C20 alcohol type sulfosuccinates, such as dioctyl sulfosuccinates and disodium laureth sulfosuccinate.

Preferably, the one or more foam boosters also provide for skin caring action and enhances the drying time of the composition while rubbing onto the skin.

A single foam booster as well as a mixture of different foam boosters may be used in the composition according to the invention.

The foam booster should further be skin compatible, i.e. not particularly irritating to the skin, eyes or mucosal tissues, and stable in the presence of alcohol.

Surprisingly, several surfactants which are known in the art as good foaming surfactants are not particularly effective as foam boosters in the compositions of this invention. Foam boosters that do not provide a stable foam according to this invention in the presence of an alcohol are highly foaming surfactants such as dodecyl benzene sulphonic acid, several high foaming alkylpolyglucosides and sodium lauryl ether sulphate.

The composition of the invention may advantageously produce a microbial count reductions in a EN 1276 suspension test on E. coli, S. aureus, E. hirae and P. aeruginosa of at least log 5 in 60 seconds, preferably in 30 seconds.

In order to ensure sufficient disinfection capacity at lower alcohol levels, several additional compounds may be present in the composition.

An acid is preferably present in the composition of the invention. The acid may be an inorganic or an organic acid. Mixtures of acids are also contemplated as being useful. The acid is present in an effective amount to establish a targeted pH range for a composition according to the invention. While any number of acids may be used, the acid preferably is a single acid. A particularly preferred acid is a carboxylic acid, such as citric acid, glycolic acid, lactic acid, succinic acid, salicylic acid, tartaric acid, sulfamic acid, glutaric acid, 2-furan carboxylic acid, or benzoic acid.

The composition of the invention has a pH of 3-8, preferably a pH of 3-7, more preferably 4-6, most preferably 4-5. Such pH may be maintained, for example, by the inclusion of one or more acids as described herein or a suitable pH buffer.

In order to improve the skin conditioning properties of the composition, the composition further may comprise one or more skin conditioning agents.

For instance, the composition may comprise an emollient or moisturizer, such as glycerol, polyglycerol, glycerides, carnithine, sorbitol, castor oil, aloe vera, allantoin, lanolin and its derivatives, acetamide MEA, agarose, ammonium lactate, arginine PCA, benzyl hyaluronate, carboxymethyl chitosan succinamide, chitosan PCA, cetyl alcohol, corn glycerides, diglycerin, dimethyl imidazolidinone, erythritol, fructose, glucamine, glucose, glucose glutamate, glucuronic acid, glutamic acid, glycereth-7, glycereth-12, glycereth-20, glycereth-26, glycereth-31, hydrogenated honey, hydrogenated starch hydrolysate, hydrolyzed corn starch, hydrolyzed wheat starch, hydroxyethyl palmityl oxyhydroxypropyl palmitamide, hydroxyethyl sorbitol, inulin, lactamide, lactamide DEA, lactamide MEA, lactic acid, lactitol, lactose, lactulose, lysine PCA, magnesium PCA, maltitol, maltose, manganese pea, mannitol, methoxypropylgluconamide, methyl gluceth-10, methyl gluceth-20, PCA, PEG-10 propylene glycol, polyamino sugar condensate, polyglucorinic acid, polyglycerin-3, polyglycerin-4, polyglycerin-6, polyglycerin-10, potassium lactate, potassium PCA, propylene-glycol, propylene glycol citrate, saccharide hydrolysate, saccharide isomerate, sodium aspartate, sodium glucuronate, sodium hyaluronate crosspolymer, sodium lactate, sodium malate, sodium PCA, sodium polyaspartate, sorbityl silanediol, tea-lactate, tea-PCA, urea, xylitol, xylose, and a combination of two or more of these emollients.

The composition may also comprise a skin conditioner, such as behentrimonium chloride, cetrimonium chloride, stearalkonium chloride, behenoyl PG-trimonium chloride.

Preferably, the skin conditioning agent is used in a concentration of 0.01-2%, more preferably 0.02-1%.

When choosing a foam booster, it may be advantageously taken into account that the foam booster also has skin conditioning properties.

A foam booster with "skin conditioning" properties is able to improve skin moisturization and provide a good after-use feeling of the skin, i.e. has an emollient or softening effect on the skin and prevents dryness of the skin with repeated use. Examples of such foam boosters are non-ionic surfactants with a good wetting capacity which are found within the alkoxylated fatty acid alcohols and alkoxylated fatty oils as described above.

To avoid build up and stickiness of the composition of the invention on the skin, it is preferred that various characteristics of surfactants are combined in as few a number of surfactants as possible, more preferably applied in the lowest feasible concentrations. Therefore, in one embodiment of the invention, foam boosting, wetting and skin conditioning functionalities are included in one compound.

Important is that the composition of the invention can be repeatedly applied, in order to comply with the applicable disinfection standards, without any further rinsing, and does not leave substantial amounts of unwanted residues.

Therefore, it is important that the composition of the invention is as skin conditioning and skin friendly as possible, i.e. that the composition not only has a skin conditioning effect, but also does not produce any form of skin irritation, allergic reaction, and most importantly does not produce any sticky residue on the skin.

Production of sticky residue may occur with many surfactants which may be generally considered for use on the skin by those skilled in the art. It is important to select a surfactant or surfactant combination which displays the above characteristics. The examples stated in this invention exemplify various compositions that advantageously possess both good skin conditioning properties as well as a good skin compatibility.

Optionally, the composition may comprise 0.01-0.5% of a complexing agent to stabilize the hydrogen peroxide in the composition and to complex or sequester interfering metal ions. Such a hydrogen peroxide stabilizer is preferably a cation sequestering agent and may be chosen from ethylenediaminetetraacetic acid (EDTA), diethylenetriaminepentaacetic acid (DTPA), N-(hydroxyethyl)-ethylenediaminetriacetic acid (HEDTA), nitrilotriacetic acid (NTA), 2-hydroxyethyliminodiacetic acid (HEIDA), and salts thereof or more preferably is chosen from acetanilide, trisodium ethylenediamine disuccinate, phosphonic acid derivatives having 1 to 5 phosphonic acid groups, for instance a Dequest phosphonate (Solutia), 1-hydroxyethylidene-1,1-diphosphonic acid (HEDP), amino tri(methylene phosphonic acid), diethylenetriamine-penta(methylene phosphonic acid), 2-hydroxy ethylimino bis(methylene phosphonic acid), and ethylene diamine tetra(methylene phosphonic acid).

Furthermore, the composition may comprise fragrances, coloring agents and/or solubilizers. In compositions which include a fragrance, it is frequently desirable to include a fragrance solubilizer which assists in the dispersion, dissolution or mixing of the fragrance constituent in an aqueous base. These solubilizers include known compounds, such as condensates of 2 to 30 moles of ethylene oxide with sorbitan mono- and tri-C10-C20 alkanoic acid esters, which are also known as non-ionic surfactants.

Additionally, specific cleaning agents or detergents may optionally be added. Especially when the composition is being applied in a wipe (towellete), the addition of cleaning agents is preferable in order to have the composition clean and disinfect in one. This is particularly useful when the composition is used to clean and disinfect hands.

The balance of the composition consists of deionized water, preferably with a conductivity of less than 5 microSiemens. It will be appreciated that the lower the deionized water conductivity, the longer the shelf life of the product.

In a preferred embodiment, the composition of the invention is essentially free of quaternary ammonium compounds, phenolic compounds, biguanide compounds and/or antimicrobial essential oils. Essentially free means that the composition comprises less than 0.05% (w/w), preferably less than 0.01% (w/w) of the compound.

In a second aspect, the invention provides the use of the composition of the first aspect for disinfection and/or sanitization of skin and/or mucous membranes, preferably for disinfection and/or sanitization of hands and arms. The invention provides use of the composition in a foam dispenser, to produce an instant foam that is easily applicable onto skin or mucous membranes.

Next to being suitable for use on the skin, the composition also may be used for other disinfecting and/or sanitizing purposes, such as for use on hard surfaces.

Use of the composition results in the eradication of, or in reducing the amount of, gram positive or gram negative organisms, fungi, yeasts and enveloped and non-enveloped viruses.

Often the prescription in healthcare settings is to use a certain specific amount of a disinfectant liquid on the skin and remain the surface wetted to allow for a desired contact time. The use of the composition of the invention as a foam advantageously allows a very precise volume to be produced and applied to the skin, a good spreading of the composition on the skin and a precise maintenance of the appropriate contact time, which is important to fulfil disinfection compliance. Furthermore, because the alcohol level is relatively low, the composition of the invention is does not evaporate before reaching the required contact time and is less flammable, increasing its practical utility and avoiding usage and storage precautions.

In order to achieve proper disinfection compliance, the product should further allow for repeated usage during the day, at least after each patient or specific activity which may cause infection of the hands. The composition of the invention is advantageously used repeatedly without significant build up of components of the composition on the skin. Practitioners will therefore be stimulated to use the composition of the invention, which also favours disinfection compliance.

### EXAMPLES

Biocidal activity of the exemplified compositions was tested using a controlled bactericidal suspension test conform European Norm for chemical disinfectants and antiseptics EN 1276 (EN 1276: Quantitative suspension test for the evaluation of bactericidal activity of chemical disinfectants and antiseptics used in food, industrial, domestic, and institutional areas: test method and requirements). One ml of a test suspension containing about 10⁸ cfu of the test microorganism per ml is added to 8 ml of the composition to be tested, and 1 ml milli-Q water is added. A clean and dirty condition is simulated by adding bovine albumin serum (0.3% and 3.0% respectively). After 30 seconds or 1 minute contact time, the amount of viable bacteria was determined. The EN 1276 norm prescribes a log 5 reduction in viable count after a contact time of 5 minutes.

### Explanation of Compounds

| | |
|---|---|
| Ethanol Denatured | Commercially available, denatured with a skin compatible and low odor compound |
| Hydrogen Peroxide | Commercially available (Akzo Nobel, Solvay) |
| Dehydol LT 7 | C12-C18 7 moles EO fatty alcohol (Cognis) |
| Tego Betaine F | Cocamidopropyl betaine (Degussa) |
| Tego Betaine F 50 | Cocamidopropyl betaine (Degussa) |
| Rewoteric AMC | Sodium cocoamphoacetate (Degussa) |
| Natrulon H6 | C6 Polyglycerol (Lonza Inc.) |
| Natrulon H10 | C10 polyglycerol(Lonza Inc.) |
| Rewopol SB FA 30 | Disodium Laureth Sulfosuccinate (Degussa) |
| Natrulon RC 50 DG | L-Carnithine and Decaglycerol mixture (Lonza Inc.) |
| Eumulgin HRE 40 | PEG-40 Hydrogenated Castor Oil (Cognis) |
| Citric Acid | Commercially generally available (various providers) |
| Benzoic Acid | Commercially generally available (various providers) |
| Barlox 10s | N,N-dimethyldecylamine N-oxide (Lonza Inc.) |
| Barlox 12 | Coco alkyldimethyl amine, N-oxide (Lonza Inc.) |
| Tween 80 | Polysorbate 80 (Uniqema) |
| EDTA | Ethylene Diamine Tetra-acetic Acid |
| Dequest SPE 9505 | Diethylene triamine penta (methylene phosphonic acid) (Solutia) |

### Composition I

38% Ethanol
1.5% Hydrogen Peroxide
0.6% Dehydol LT 7
0.3% Natrulon H-6
0.2% Benzoic Acid
0.1% Barlox 12 (Cocoamine Oxide)
0.1% EDTA
pH ca. 4.5

### Log 10 reduction according to EN 1276 at 1 minute contact time

| | Clean | Dirty |
|---|---|---|
| E. coli ATCC 25922 | > 6.2 | > 6.2 |
| Pseudomonas aeruginosa ATCC 15442 | > 6.2 | > 6.2 |
| Enterococcus hirae ATCC 10541 | > 5.6 | > 5.6 |
| Staphylcoccus aureus ATCC 6538 | > 5.6 | > 5.6 |

### Composition II

35% Ethanol
1% Hydrogen peroxide
0.7% Dehydol LT 7
0.15% Barlox 12
0.2% Benzoic Acid
pH ca. 5

### Log 10 reduction according to EN 1276 at 1 minute contact time

| | Clean | Dirty |
|---|---|---|
| E. coli ATCC 25922 | > 6.2 | > 6.2 |
| Pseudomonas aeruginosa ATCC 15442 | > 6.2 | > 6.2 |
| Enterococcus hirae ATCC 10541 | > 5.6 | > 5.6 |
| Staphylcoccus aureus ATCC 6538 | > 5.6 | > 5.6 |

### Composition III

40% Ethanol
0.7% Hydrogen peroxide
0.6% Dehydol LT 7
0.1% Barlox 10s (Decylamine Oxide)
0.2% Benzoic Acid
0.1% Dequest
pH ca. 4.6

### Log 10 reduction according to EN 1276 at 30 seconds contact time

| | Clean | Dirty |
|---|---|---|
| E. coli ATCC 25922 | 5 | 5 |
| Pseudomonas aeruginosa ATCC 15442 | 5 | 5 |
| Enterococcus hirae ATCC 10541 | 5 | 5 |
| Staphylcoccus aureus ATCC 6538 | 5 | 5 |

The compositions also provide for a broad spectrum antimicrobial activity, evidenced by a European fungicidal suspension test (EN 1275 Chemical disinfectants and antiseptics - Quantitative suspension test for the evaluation of basic fungicidal activity or basis yeasticidal activity of chemical disinfectants and antiseptics - Test method and requirements (phase 1) and EN 1650 Chemical disinfectants and antiseptics - Quantitative suspension test for the evaluation of fungicidal activity of chemical disinfectants and antiseptics used in food, industrial, domestic, and institutional areas - Test method and requirements (phase 2, step 1)

### Composition IV

33% Ethanol
1.5% Hydrogen peroxide
0.3% Eumulgin HRE 40 (PEG-40 Hydrogenated Castor Oil)
0.5% Protelan LS 9011 (Sodium Lauryl Sarcosinate)
0.2% Salicylic Acid
pH ca. 4.2

### Log 10 reductions in European norm suspension tests:

| | |
|---|---|
| EN 1275 | 5 minutes |
| Candida Albicans ATCC 12031 | > log 4 |
| EN 1275 and EN 1650 (clean & dirty) | 15 minutes |
| Candida Albicans ATCC 12031 | > 5 |
| Aspergillus Niger ATCC 16404 | > 5 |

### Composition V

20% Ethanol
10% Isoproyl Alcohol
1% Hydrogen peroxide
0.2% Tween 80
0.2% Tego Betain L7 (cocoamidoproyl betaine)
0.2% Lactic Acid
0.1% Dequest
pH ca. 5

### Log 10 reduction according to EN 1276 at 30 seconds contact time

| | Clean | Dirty |
|---|---|---|
| E. coli ATCC 25922 | 5 | 5 |
| Pseudomonas aeruginosa ATCC 15442 | 5 | 5 |
| Enterococcus hirae ATCC 10541 | 5 | 5 |
| Staphylcoccus aureus ATCC 6538 | 5 | 5 |

Besides good biocidal activity, the compositions I to V as disclosed above provide a stable foam when dispensed from an S10 or L11 dispenser (Rexam Airspray).

Table 1 shows various compositions with a good foam quality and slightly differing skin after-feel. Weight percentages are net weight percentages of the ingredients based on total weight of the composition.

The reductions in E. coli and Staphylococcus aureus were tested in an EN 1276 suspension test and show the antimicrobial efficacy of the composition on respectively gram negative and gram positive bacteria.

**Table 1**

| | **1** | **2** | **3** | **4** | **5** | **6** |
|---|---|---|---|---|---|---|
| Ethanol | 35% | 36% | 38% | 40% | 42% | 36% |
| H2O2 | 0.5% | 0.7% | 0.3% | 0.3% | 0.6% | 1% |
| Dehydol LT 7 | 0.2% | 0.3% | 0.5% | 0.6% | 0.7% | 0.3% |
| Barlox 10s | | | | 0.1% | | |
| Barlox 12 | 0.1% | | 0.1% | | | |
| Tego Betaine F | | | | | 0.10 % | |
| Tego Betaine F 50 | | | | | | |
| Rewopol SB FA 30 | | | | | | |
| Rewoteric AMC | | | | | | |
| Natrulon RC 50 DG | 0.3% | | 0.3% | | | |
| Natrulon H6 | | | | | | 0.2% |
| Natrulon H10 | | | | 0.2% | | |
| Glycerol | | 0.5% | | | | |
| Citric Acid | | | | 0.1% | 0.1% | 0.1% |
| Benzoic Acid | 0.3% | 0.4% | 0.3% | | | |
| EDTA | 0.2% | 0.1% | | 0.2% | | |
| Dequest SPE 9505 | | | 0.1% | | 0.1% | |
| | | | | | | |
| E. coli | >5 | >5 | >5 | >5 | >5 | >5 |
| Staph. Aureus | >5 | >5 | >5 | >5 | >5 | >5 |
| | | | | | | |
| Foam Quality | ++ | ++ | ++ | ++ | ++ | ++ |
| Skin Feel | ++ | ++ | ++ | + | +/- | ++ |
| | | | | | | |

| | **7** | **8** | **9** | **10** | **11** | **12** |
|---|---|---|---|---|---|---|
| Ethanol | 36% | 36% | 36% | 36% | 36% | 36% |
| H2O2 | 1% | 1% | 1% | 1% | 1% | 1% |
| Dehydol LT 7 | | | | | | |
| Barlox 10s | 0.2% | | | | | |
| Barlox 12 | | 0.2% | | | | |
| Tego Betaine F | | | 0.2% | | | |
| Tego Betaine F 50 | | | | 0.2% | | |
| Rewopol SB FA 30 | | | | | 0.2% | |
| Rewoteric AMC | | | | | | 0.2% |
| Natrulon RC 50 DG | | | | | | |
| Natrulon H6 | | | | | | |
| Natrulon H10 | | | | | | |
| Glycerol | | | | | | |
| Citric Acid | | | | | | |
| Benzoic Acid | 0.3% | 0.3% | 0.3% | 0.3% | 0.3% | 0.3% |
| EDTA | | | | | | |
| Dequest SPE 9505 | | | | | | |
| | | | | | | |
| E. coli | >5 | >5 | >5 | >5 | >5 | >5 |
| Staph. Aureus | >5 | >5 | >5 | >5 | >5 | >5 |
| | | | | | | |
| Foam Quality | + | ++ | ++ | ++ | + | + |
| Skin Feel | + | ++ | + | + | +/- | +/- |

## Claims

1. An aqueous composition, wherein, when the composition is mixed with air using a foam dispenser, the mixture of the composition and air produces a foam, the composition being an aqueous solution having a pH in the range of 3-8 and comprising:
0.1-5% (w/w) hydrogen peroxide,
26-50% (w/w) of a C2-C6 alcohol, and
0.01-2% (w/w) of a foam booster chosen from at least one of i) an alkoxylated non-ionic surfactant, ii) a nitrogen containing amphoteric or non-ionic surfactant, iii) a linear C12-C24 alpha olefin sulfonate, and iv) a C12-C20 sulfosuccinate,
the balance of the composition being deionized water.

2. The composition of claim 1, wherein the foam booster is chosen from at least one of i) an alkoxylated non-ionic surfactant, ii) a nitrogen containing amphoteric or non-ionic surfactant.

3. The composition of claim 1 or 2, which is essentially free of phenolic compounds, quaternary ammonium compounds, biguanide compounds and/or antimicrobial essential oils.

4. The composition of any one of the claims 1 to 3, wherein the alkoxylated non-ionic surfactant i) is chosen from at least one of an alkoxylated C8-C30 fatty alcohol, C8-C30 fatty acid and C8-C30 fatty oil, all with a degree of alkoxylation of minimal 6, and a polysorbate.

5. The composition of claim 4, wherein the alkoxylated fatty alcohol contains a fatty alcohol component that is a C10-C20 primary and/or secondary alcohol, preferably a linear C10-C20 primary and/or secondary alcohol.

6. The composition of claim 5, wherein the alkoxylated fatty oil is a hydrogenated C10-C20 fatty oil, preferably having a degree of alkoxylation of at least 20.

7. The composition of any on of the claims 1-6,
wherein the alkoxylated non-ionic surfactant i) is an ethoxylated surfactant.

8. The composition of any one of the claims 1 to 7, wherein the nitrogen containing amphoteric or non-ionic surfactant ii) is chosen from at least one of an Amine Oxide, a Betaine, an Amide, a Hydroxysultaine, and/or an Imidazoline derivative.

9. The composition of any one of claims 1-8 further comprising 0.01-2% (w/w) of a skin conditioning agent, preferably at least one of glycerol, polyglycerol, glycerides, carnithine, sorbitol, castor oil, aloe vera, allantoin, lanolin and its derivatives, and cetyl alcohol.

10. The composition of any one of claims 1-9,
wherein the foam booster, next to foam boosting, also includes wetting and skin conditioning functionalities.

11. The composition of any one of claims 1-10 further comprising a carboxylic acid comprising a cyclic carboxylic acid.

12. Use of the composition of any one of the preceding claims for disinfection and/or sanitization of the skin or mucous membranes and/or of hard surfaces.

13. Use of the composition of any one of the claims 1-11 in a foam dispenser, in order to produce a foam.

14. Method for disinfection and/or sanitization of the skin or mucous membranes comprising applying the composition of any one of the claims 1-11 as a foam onto the skin or mucous membranes.

15. Method for producing a foam comprising dispensing the composition of any one of the claims 1-11 from a foam dispenser.

## Patentansprüche

1. Eine wässrige Zusammensetzung, wobei, wenn die Zusammensetzung mittels eines Schaumspenders mit Luft gemischt wird, die Mischung aus der Zusammensetzung und Luft einen Schaum erzeugt, wobei die Zusammensetzung eine wässrige Lösung mit einem pH-Wert im Bereich von 3 bis 8 ist und umfasst:
0,1% (w/w) bis 5% (w/w) Wasserstoffperoxid,
26% (w/w) bis 50% (w/w) eines C2-C6-Alkohols,
0,01% (w/w) bis 2% (w/w) eines Schaumverstärkers, der aus mindestens einem von i) einem alkoxylierten nichtionischen Tensid, ii) einem Stickstoff-enthaltenden amphoteren oder nichtionischen Tensid, iii) einem linearen C12-C24-Alphaolefinsulfonat, und iv) einem C12-C20-Sulfosuccinat ausgewählt ist,
wobei der Rest der Zusammensetzung entionisiertes Wasser ist.

2. Die Zusammensetzung gemäß Anspruch 1, wobei der Schaumverstärker aus mindestens einem von i) einem alkoxylierten nichtionischen Tensid, ii) einem Stickstoff-enthaltenden amphoteren oder nichtionischen Tensid enthält, ausgewählt ist.

3. Die Zusammensetzung gemäß Anspruch 1 oder 2, die im Wesentlichen frei von phenolischen Verbindungen, quaternären Ammoniumverbindungen, Biguanidverbindungen und/oder antimikrobiellen ätherischen Ölen ist.

4. Die Zusammensetzung gemäß irgendeinem der Ansprüche 1 bis 3, wobei das alkoxylierte nichtionische Tensid i) ausgewählt ist aus mindestens einem von einem alkoxylierten C8-C30-Fettalkohol, C8-C30-Fettsäure und C8-C30-Fettöl, alle mit einem Alkoxylierungsgrad von mindestens 6, und einem Polysorbat.

5. Die Zusammensetzung gemäß Anspruch 4, wobei der alkoxylierte Fettalkohol einen Fettalkoholbestandteil enthält, der ein C10-C20- primärer und/oder sekundärer Alkohol, vorzugsweise ein linearer C10-C20- primärer und/oder sekundärer Alkohol ist.

6. Die Zusammensetzung gemäß Anspruch 5, wobei das alkoxylierte Fettöl ein hydriertes C10-C20-Fettöl ist, das vorzugsweise einen Alkoxylierungsgrad von mindestens 20 hat.

7. Die Zusammensetzung gemäß irgendeinem der Ansprüche 1 bis 6, wobei das alkoxylierte nichtionische Tensid i) ein ethoxyliertes Tensid ist.

8. Die Zusammensetzung gemäß irgendeinem der Ansprüche 1 bis 7, wobei das Stickstoff-enthaltende amphotere oder nichtionische Tensid ii) aus mindestens einem von einem Aminoxid, einem Betain, einem Amid, einem Hydroxysultain und /oder einem Imidazolinderivat ausgewählt ist.

9. Die Zusammensetzung gemäß irgendeinem der Ansprüche 1 bis 8, ferner umfassend 0,01% (w/w) bis 2% (w/w) eines Hautpflegemittels, vorzugsweise mindestens einem von Glycerin, Polyglycerin, Glyceriden, Carnithin, Sorbitol, Castoröl, Aloe Vera, Allantoin, Lanolin und dessen Derivaten und Cetylalkohol.

10. Die Zusammensetzung gemäß irgendeinem der Ansprüche 1 bis 9, wobei der Schaumverstärker neben dem schaumverstärkenden auch Befeuchtungs- und Hauptpflegefunktionen besitzt.

11. Die Zusammensetzung gemäß irgendeinem der Ansprüche 1 bis 10, ferner umfassend eine Carbonsäure umfassend eine zyklische Carbonsäure.

12. Verwendung der Zusammensetzung gemäß irgendeinem der vorhergehenden Ansprüche zum Desinfizieren und/oder Reinigen der Haut oder von Schleimhäuten und/oder von harten Oberflächen.

13. Verwendung der Zusammensetzung gemäß irgendeinem der Ansprüche 1 bis 11 in einem Schaumspender, um einen Schaum zu erzeugen.

14. Verfahren zum Desinfizieren und/oder Reinigen der Haut oder von Schleimhäuten, umfassend das Aufbringen der Zusammensetzung gemäß irgendeinem der Ansprüche 1 bis 11 als einem Schaum auf die Haut oder Schleimhäute.

15. Verfahren zum Herstellen eines Schaums, umfassend das Abgeben der Zusammensetzung gemäß irgendeinem der Ansprüche 1 bis 11 aus einem Schaumspender.

## Revendications

1. Composition aqueuse, dans laquelle, lorsque la composition est mélangée avec de l'air à l'aide d'un distributeur de mousse, le mélange de la composition et de l'air produit une mousse, la composition étant une solution aqueuse ayant un pH se trouvant dans la plage allant de 3 à 8 et comprenant :
0,1-5% (w/w) de peroxyde d'hydrogène,
26-50% (w/w) d'un alcool en C2-C6, et
0,01-2% (w/w) d'un renforçateur de mousse choisi parmi au moins l'un i) d'un tensio-actif non ionique alcoxylé ii) d'un tensioactif non ionique ou amphotère contenant de l'azote, iii) d'un sulfonate d'alpha-oléfine en C12-C24 linéaire, et iv) d'un sulfosuccinate en C12-C20,
le reste de la composition étant de l'eau déionisée.

2. Composition de la revendication 1, dans laquelle le renforçateur de mousse est choisi parmi au moins l'un i) d'un tensio-actif non ionique alcoxylé ii) d'un tensioactif non ionique ou amphotère contenant de l'azote.

3. Composition de la revendication 1 ou 2, qui est essentiellement exempte de composés phénoliques, de composés d'ammonium quaternaire, de composés de biguanide et/ou d'huiles essentielles antimicrobiennes.

4. Composition de l'une quelconque des revendications 1 à 3, dans laquelle le tensioactif non ionique alcoxylé i) est choisi parmi au moins l'un(e) d'un alcool gras en C8-C30, d'un acide gras en C8-C30 et d'une huile grasse en C8-C30 alcoxylés, tous ces composés ayant un degré d'alcoxylation d'au moins 6, et du polysorbate.

5. Composition de la revendication 4, dans laquelle l'alcool gras alcoxylé contient un composant d'alcool gras qui est un alcool primaire et/ou secondaire en C10-C20, de préférence un alcool primaire et/ou secondaire en C10-C20 linéaire.

6. Composition de la revendication 5, dans laquelle l'huile grasse alcoxylée est une huile grasse en C10-C20 hydrogénée ayant de préférence un degré d'alcoxylation d'au moins 20.

7. Composition de l'une quelconque des revendications 1-6, dans laquelle le tensioactif non ionique alcoxylé i) est un tensioactif éthoxylé.

8. Composition de l'une quelconque des revendications 1 à 7, dans laquelle le tensioactif non ionique ou amphotère contenant de l'azote ii) est choisi parmi au moins l'un(e) d'un oxyde d'amine, d'une bétaïne, d'un amide, d'un hydroxysultaine, et/ou d'un dérivé d'imidazoline.

9. Composition de l'une quelconque des revendications 1-8 comprenant en outre 0,01-2% (w/w) d'un agent de conditionnement de la peau, de préférence au moins l'un(e) parmi le glycérol, polyglycérol, glycérides, carnitine, sorbitol, huile de ricin, aloe vera, allantoïne, lanoline et ses dérivés, et l'alcool cétylique.

10. Composition de l'une quelconque des revendications 1 à 9, dans laquelle le renforçateur de mousse, en plus de sa fonctionnalité de renforcement de mousse, possède également des fonctionnalités de mouillage et de conditionnement de la peau.

11. Composition de l'une quelconque des revendications 1-10, comprenant en outre un acide carboxylique comprenant un acide carboxylique cyclique.

12. Utilisation de la composition de l'une quelconque des revendications précédentes pour la désinfection et/ou l'aseptisation de la peau ou des membranes muqueuses et/ou de surfaces dures.

13. Utilisation de la composition de l'une quelconque des revendications 1-11 dans un distributeur de mousse, afin de produire une mousse.

14. Procédé de désinfection et/ou d'aseptisation de la peau ou des membranes muqueuses comprenant l'application de la composition de l'une quelconque des revendications 1-11 comme une mousse sur la peau ou les membranes muqueuses.

15. Procédé de production d'une mousse comprenant le fait de distribuer la composition de l'une quelconque des revendications 1-11 à partir d'un distributeur de mousse.
